Europäisches Patentamt

European Patent Office

Office européen des brevets

(19)

(11) EP 0 657 730 B1

(12) **FASCICULE DE BREVET EUROPEEN**

(45) Date de publication et mention
de la délivrance du brevet:
**30.09.1998 Bulletin 1998/40**

(51) Int Cl.$^6$: **G01N 15/14**, G01N 33/22

(21) Numéro de dépôt: **94402814.1**

(22) Date de dépôt: **07.12.1994**

(54) **Procédé automatique d'analyse macérale et de détermination du pouvoir réflecteur de la vitrinite dans les charbons**

Verfahren zur automatischen Maceralanalyse und zur Bestimmung des Reflexionsvermögens von Vitrinit in Kohle

Method for automatically maceral analysing and for determining reflectance of vitrinite in coals

(84) Etats contractants désignés:
**AT BE CH DE DK ES FR GB GR IE IT LI LU NL PT SE**

(30) Priorité: **10.12.1993 FR 9314884**

(43) Date de publication de la demande:
**14.06.1995 Bulletin 1995/24**

(73) Titulaire: **SOLLAC**
**92800 Puteaux (FR)**

(72) Inventeur: **Celeski, Jean-Claude**
**F-13270 Fos sur mer (FR)**

(74) Mandataire: **Polus, Camille et al**
**c/o Cabinet Lavoix**
**2, Place d'Estienne d'Orves**
**75441 Paris Cedex 09 (FR)**

(56) Documents cités:
**WO-A-83/00556**      **FR-A- 2 649 794**
**US-A- 4 030 837**      **US-A- 4 617 682**
**US-A- 5 155 546**

- **INTERNATIONAL LABORATORY., vol.15, no.7, Septembre 1985, FAIRFIELD CT US pages 78 - 85 M. MORI ET AL. 'Automatic measurement of coke microtextures'**

Printed by Jouve, 75001 PARIS (FR)

## Description

L'invention concerne un procédé qui permet de caractériser les charbons en vue de déterminer en particulier leur teneur en vitrinite.

Les modèles d'élaboration et de prévision de qualité du coke utilisent certains paramètres déduits de l'analyse pétrographique tels que le pouvoir réflecteur et la proportion ou teneur en vitrinite qui est le principal constituant des charbons. Les valeurs de ces deux paramètres permettent d'évaluer les propriétés cokéfiantes du charbon.

Les charbons entrant dans la pâte à coke ont un pouvoir réflecteur de la vitrinite qui varie de 0,5% à 1,7%, la proportion de vitrinite se situant entre 40% et 85%. Les charbons à haut pouvoir cokéfiant se caractérisent par un pouvoir réflecteur de la vitrinite qui est compris entre 1,1% et 1,3% et par une teneur élevée en vitrinite.

On contrôle de manière systématique les approvisionnements en charbon de manière à vérifier la conformité et la régularité de la qualité des matières premières dans le but de contribuer à l'élaboration d'un coke de qualité constante et de mieux évaluer le prix d'achat des charbons.

Le contrôle de la qualité des charbons s'effectue actuellement de manière manuelle. A cet effet, on utilise un échantillon qui est constitué par du charbon broyé enrobé dans de la résine ; cet échantillon est poli et il est placé dans un microscope optique dont le grossissement est par exemple de 300.

Le microscope est équipé d'un réflectomètre qui est calibré grâce à des surfaces étalons de pouvoir réflecteur connu.

On mesure le pouvoir réflecteur sur un échantillon en le balayant selon une maille régulière. La précision de la mesure est fonction du nombre de mesures réalisées.

La norme définie par les pétrographes préconise 200 mesures pour un charbon classique. Lorsque l'on veut mettre en évidence soit une pollution, soit un mélange, il est nécessaire d'échantillonner avec 300 ou 400 prélèvements.

L'analyse macérale consiste à déterminer la proportion des principaux constituants en balayant l'échantillon selon le principe décrit ci-dessus. L'opérateur définit le macéral qui se trouve au centre du réticule et totalise sa fréquence d'apparition. La norme préconise 500 prélèvements pour estimer la proportion des différents macéraux, à savoir essentiellement l'exinite, la vitrinite, la fusinite, la semi-fusinite et l'inertinite.

La méthode manuelle de la mesure du pouvoir réflecteur moyen et de l'analyse macérale nécessite de trois à six heures d'analyse selon la difficulté du charbon.

Cette durée d'analyse est importante et nécessite une présence permanente de l'opérateur ; de plus la méthode est contraignante. La précision des mesures est faible surtout en ce qui concerne l'analyse macérale.

C'est pourquoi la présente invention propose de fournir un procédé automatique qui permette de déterminer la valeur du pouvoir réflecteur de la vitrinite et de réaliser l'analyse macérale avec une précision améliorée et un temps de mesure réduit.

A cet effet, l'invention a pour objet un procédé d'analyse macérale du charbon en vue de déterminer sa teneur en vitrinite, dans lequel on utilise un échantillon constitué par du charbon broyé enrobé dans de la résine, on explore la surface de l'échantillon au moyen d'un microscope en déplaçant l'échantillon pour obtenir des champs successifs dans le microscope et l'on mesure le pouvoir réflecteur de la vitrinite. Un tel procédé est connu du document US-A-4 617 682. Le procédé est caractérisé par les étapes suivantes:

- pour chaque champ du microscope, on réalise une image numérisée à niveaux de gris ;
- sur chaque image, on effectue une mesure de seuillage pour éliminer les champs contenant une quantité de résine supérieure à un seuil donné (80%) ;
- sur les champs éliminés, on ne réalise pas de mesures, car ces champs contiennent trop de défauts (polissage, préparation). Leur élimination ne biaise pas les mesures ;
- pour chaque champ non éliminé, on effectue l'analyse suivante :

  a) on détermine la distribution des niveaux de gris ;
  b) on détermine les gradients des niveaux de gris;
  c) on réalise une image binaire par seuillage des gradients de gris de manière à déterminer des zones de vitrinite ;
  d) on détermine la distribution des niveaux de gris ou densitométrie des zones de vitrinite obtenues ; et
  e) on calcule le pouvoir réflecteur de la vitrinite.

Selon une autre caractéristique de l'invention, le pouvoir réflecteur de la vitrinite est calculé en déterminant le niveau de gris moyen pour l'ensemble des zones de vitrinite de l'échantillon.

Selon encore une autre caractéristique de l'invention, le pouvoir réflecteur de la vitrinite est calculé en déterminant le niveau de gris moyen de chaque zone de vitrinite.

D'autres caractéristiques et avantages de l'invention ressortiront de la description qui suit d'un exemple de réalisation de l'invention, faite en se référant aux annexés sur lesquels :

- la figure 1 est un diagramme linéaire du pouvoir réflecteur d'un échantillon du charbon ;
- la figure 2 est un diagramme de la distribution du pouvoir réflecteur ;
- la figure 3 est un diagramme de densitométrie sur une image numérisée en niveaux de gris avec correction de l'effet de la porosité de la résine ;
- la figure 4 illustre une opération de filtrage des images obtenues ;
- les figures 5 et 6 sont des diagrammes de distribution permettant de comparer les résultats du procédé selon l'invention avec les résultats obtenus par le procédé manuel de type connu ;
- la figure 7 est un autre diagramme de comparaison du procédé selon l'invention avec le programme manuel de type connu ;
- la figure 8 est un diagramme de distribution dans lequel on a effectué une correction des effets de la porosité pour des mesures obtenues selon le procédé selon l'invention et selon le procédé manuel de type connu ;
- la figure 9 est un diagramme de distribution permettant de mettre en évidence les autres constituants du charbon ;
- la figure 10 est une représentation schématique d'un dispositif pour la mise en oeuvre du procédé selon l'invention ;
- les figures 11A à 11C illustrent le traitement effectué dans le dispositif de la figure 10 ; et
- la figure 12 est une courbe d'étalonnage qui représente la corrélation entre le pouvoir réflecteur connu de surfaces étalons et le niveau de gris moyen.

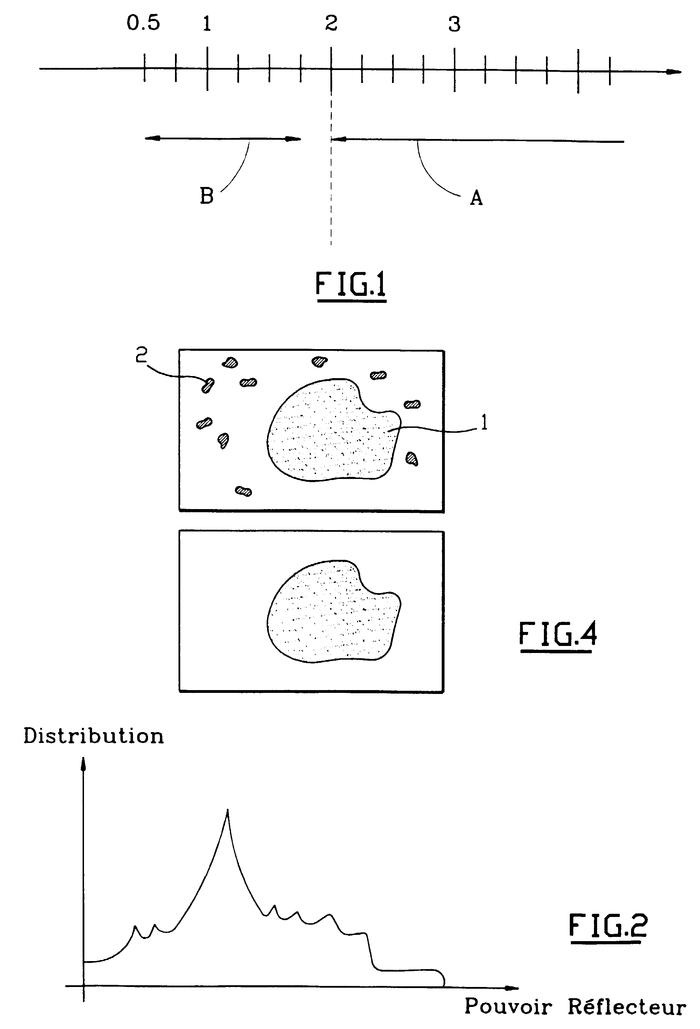

On a représenté sur la figure 1 un diagramme linéaire du pouvoir réflecteur mesuré sur un échantillon de charbon. Lorsque le pouvoir réflecteur est supérieur à 2 dans la zone A, il s'agit d'un anthracite, c'est-à-dire d'un charbon de haut rang qui ne peut pas se cokéfier.

La vitrinite est de couleur claire et présente des plaques importantes présentant un pouvoir réflecteur se situant dans la zone B, c'est-à-dire compris entre 0,5 et 1,7 pour les charbons cokéfiants. Il s'agit là de charbons utilisés pour la fabrication de coke.

On a représenté dans le tableau ci-dessous la classification des charbons en fonction de leur pouvoir réflecteur.

## CLASSIFICATION DES CHARBONS

| Tourbe | Lignite | Flambants | Charbons cokéfiants | Anthracites |
|---|---|---|---|---|
| | | | Pouvoir réflecteur de la vitrinite | |
| | ← | 0,5 | 1,7 → | |
| PR moyen très faible | | | | PR moyen très élevé |

Un charbon cokéfiant se compose dans l'ordre croissant des pouvoirs réflecteurs des éléments suivants: exinite, vitrinite, semi-fusinite, fusinite et inertinite.

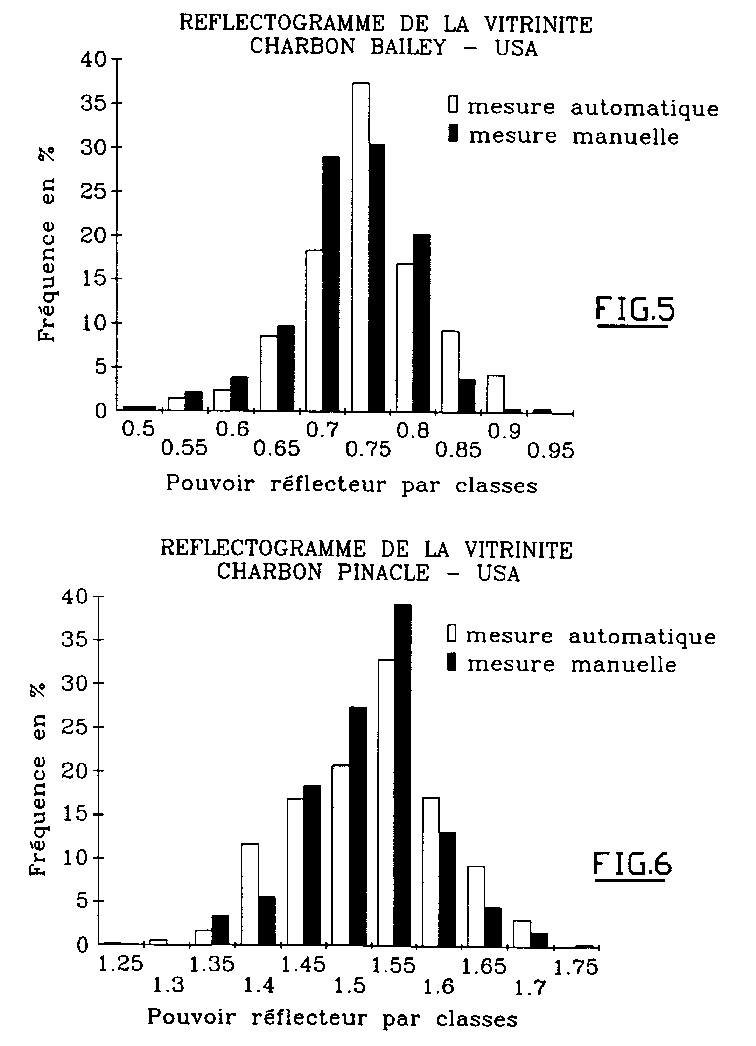

Les figures 2 et 4 montrent les facies de la vitrinite et des autres composants.

Dans le procédé connu, de type manuel, d'analyse macérale des charbons, on utilise un microscope équipé d'un réflectomètre calibré.

La détermination de la proportion des différents macéraux est basée sur l'expérience de l'opérateur qui relève à la croisée du réticule la nature du macéral. L'opérateur s'aide parfois en réalisant une mesure ponctuelle du pouvoir réflecteur pour distinguer les semi-fusinites de la vitrinite.

Ce pouvoir réflecteur est le plus faible pour l'exinite qui correspond à une proportion de 0 à 3% en masse. Dans l'ordre croissant on trouve ensuite la vitrinite dont la proportion est comprise entre 45% et 85% en masse. On trouve ensuite la semi-fusinite et la fusinite qui correspondent ensemble à une proportion comprise entre 15% et 45%. Il existe également l'inertinite, avec un pouvoir réflecteur très élevé dont la proportion est inférieure à 10%.

On voit sur la figure 2 un diagramme indiquant la distribution ou la fréquence en fonction du pouvoir réflecteur mesuré. Cette courbe permet de mesurer la qualité du charbon et de déterminer le pourcentage de la teneur en vitrinite.

La présente invention concerne un procédé d'analyse macérale automatique qui est basé sur l'observation d'un échantillon préparé dans les mêmes conditions que pour le procédé manuel de type connu.

Dans ce procédé, on utilise également un microscope et l'on effectue des opérations préliminaires de calibrage.

Tout d'abord, en utilisant une surface étalon dont le pouvoir réflecteur est connu, on effectue manuellement le réglage de l'éclairage du microscope.

Par ailleurs, on calcule la valeur d'un coefficient K pour chaque point-image de l'image numérisée ; ce coefficient K permet d'effectuer une correction automatique de l'homogénéité de manière à obtenir toujours la même valeur de gris moyen (densitométrie) sur la surface étalon.

La composition macérale est calculée de la manière suivante :

$$\% \text{ vitrinite} = \frac{\text{nombre de points relevés dans la vitrinite}}{\text{nombre total de points (500)}}$$

idem pour les autres macéraux.

En effet, l'image acquise sur un microscope optique à l'aide d'une caméra-vidéo présente souvent un défaut d'homogénéité qui est lié au réglage de l'éclairage ou à la caméra.

Pour corriger cette homogénéité, on utilise donc une image de référence acquise dans les mêmes conditions de travail sur la surface étalon qui est uniforme en niveaux de gris.

Cette image intègre les différents défauts d'uniformité ; à partir de ces valeurs différentes de niveaux de gris, on construit une matrice de la taille des images à traiter en calculant le coefficient de correction K pour chacun des points-image selon l'équation suivante :

$$K_{ij} = \frac{NG_{max}}{NG_{ij}}$$

dans laquelle $NG_{ij}$ est le niveau de gris au point $ij$ et $NG_{max}$ est le niveau de gris maximal de l'image.

La correction va consister à modifier le niveau de gris de chaque point-image proportionnellement à son coefficient K suivant le mode de calcul suivant :

$$\text{IMAGE CORRIGEE}_{ij} = \frac{K_{ij} \ \text{IMAGE}_{ij}}{SUP(K_{ij})}$$

dans laquelle $\text{IMAGE}_{ij}$ est l'image à traiter et $SUP(K_{ij})$ la valeur maximale des coefficients K.

Cette correction est particulièrement importante dans le cadre de cette application puisque l'on caractérise le pouvoir réflecteur d'un des constituants en mesurant et en mettant en évidence de faibles variations du pouvoir réflecteur.

Le but de la correction d'homogénéité est d'éviter que pour une même zone de l'échantillon, on obtienne des valeurs différentes du pouvoir réflecteur suivant la position dans le champ de la caméra.

Après ces opérations préliminaires de calibrage, l'opérateur met en place l'échantillon à analyser et saisit sur un dispositif d'enregistrement de données les références de cet échantillon.

Le reste du procédé d'analyse est entièrement automatique. Les différentes opérations sont les suivantes:

- déplacement de la platine supportant l'échantillon;
- mise au point automatique ;
- acquisition d'une image ;
- traitement de l'image ;
- mesures de densitométrie.

La dernière opération n'est effectuée que pour des images non éliminées.

L'acquisition de l'image est réalisée avec un microscope équipé d'une caméra fournissant une image numérisée à niveaux de gris. On peut par exemple utiliser une image numérisée dans laquelle les niveaux de gris varient de 0 à 255, c'est-à-dire qu'ils sont codés sur un octet.

La première opération du traitement automatique de l'image est destinée à éliminer les images correspondant à des champs du microscope pour lesquels la proportion de résine est très importante, par exemple supérieure à 80%.

A cet effet, on utilise un procédé de seuillage en analysant l'image point par point et en créant une image binaire, dans laquelle chaque pixel ne peut prendre que la valeur 0 ou 1.

Cette opération de seuillage est basée sur le fait les nuances de gris correspondant à la résine sont assez semblables sur chaque champ et chaque échantillon; l'observation d'un grand nombre d'opérations a permis de fixer la gamme de niveaux de gris correspondant à la résine entre 0 et 70 dans le cas où l'image est codée sur un octet, c'est-à-dire quand les niveaux de gris varient entre 0 et 255.

Dans ce cas, on crée une image binaire dans laquelle, pour chaque point-image, la valeur est égale à 1 si le pouvoir réflecteur est inférieur ou égal à 70 et à 0 si ce pouvoir réflecteur est supérieur à 70.

On détermine ensuite la proportion de résine d'enrobage, c'est-à-dire le rapport de la somme des valeurs binaires de l'ensemble des points-image sur le nombre total de ces points. Si cette proportion est supérieure ou égale à 80%, on élimine l'image correspondante et l'on déplace l'échantillon pour acquérir un autre champ.

Pour tous les champs ou images non éliminés, on effectue ensuite les phases suivantes.

On effectue tout d'abord une correction de l'homogénéité en affectant à chaque point-image le facteur de correction K qui a été défini plus haut.

On effectue ensuite une mesure de la distribution des niveaux de gris de manière à obtenir une densitométrie globale du charbon.

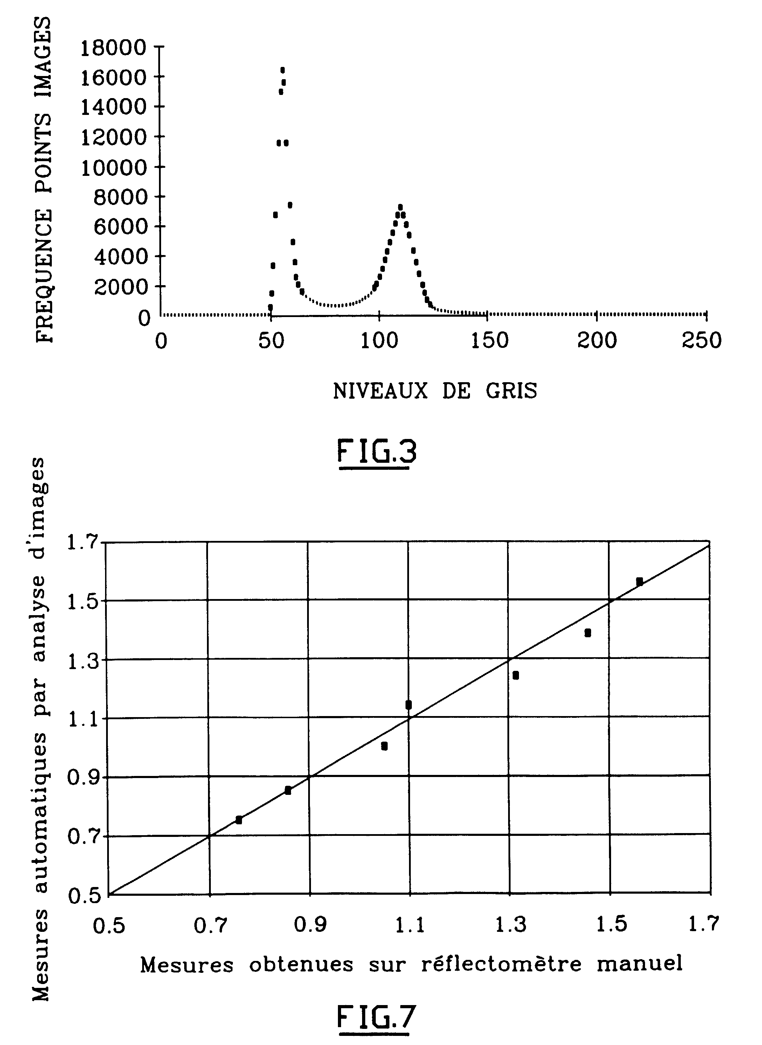

La densitométrie est définie comme étant la distribution des niveaux de gris dans une image et, plus précisément, elle représente le nombre de points-image correspondant à chaque niveau de gris. La courbe représentée sur la figure 3 représente un exemple de distribution ou de densitométrie sur une image numérisée en niveaux de gris. On a porté en abscisses les valeurs des niveaux de gris et en ordonnées la fréquence d'apparition des points-image présentant le niveau de gris correspondant.

On effectue ensuite une phase d'analyse des gradients de gris. En effet, la vitrinite est le composant des charbons qui présente la plus grande homogénéité, c'est-à-dire les plus grandes surfaces présentant très peu de variations du niveau de gris.

C'est pourquoi la mise évidence des zones homogènes de vitrinite est basée sur l'analyse des gradients de gris de l'image numérisée ayant subi la correction d'homogénéité.

Le gradient d'une image digitalisée est représenté par la formule :

$$g(x) = \frac{1}{n} \sum_{i=1}^{n} \left| f(x) - f(x_i) \right|$$

dans laquelle n est le nombre de points voisins du point-image considéré, f(x) est le niveau de gris au point-image considéré et $f(x_i)$ est la valeur du niveau de gris pour chacun des points voisins.

Le résultat du filtrage par analyse des gradients est constitué par une nouvelle image numérisée dont les niveaux de gris correspondent aux variations mesurées entre chaque point et ses voisins.

Une zone où chaque point a une même valeur de gris (uniformité parfaite) donnera une valeur de gradient égale à 0 pour chaque point-image. Le plus fort gradient pouvant exister dans une image codée sur un octet est donc égal à 255 ce qui correspond à un point-image dont la valeur de gris est égale à 0 et pour lequel les points voisins ont une valeur de gris égale à 255.

Dans le cas d'une trame carrée, chaque point-image possède huit points voisins.

L'analyse de différents charbons a permis de déterminer la valeur critique du gradient qui caractérise l'uniformité de gris de la vitrinite dans les conditions de mesures. Cette valeur est égale à 20 pour une image numérisée codée sur un octet.

On réalise alors une image binaire par seuillage des niveaux de gris des gradients. Si la valeur du gradient d'un point-image est supérieure à 20, sa valeur binaire est égale à 1 et cette valeur binaire est égale à 0 pour les autres points-images. On obtient une image binaire comme représentée en haut de la figure 4. Cette image binaire représente l'ensemble des points pour lesquels le gradient a une valeur comprise entre 21 et 255. On peut voir une zone homogène 1 de surface importante et des zones de très faibles surfaces 2.

Les opérations morphologiques telles que l'érosion sont des procédures connues dans les systèmes d'analyses d'images. Elles sont basées sur les principes de la morphologie mathématique.

L'étape suivante consiste à réaliser un filtrage morphologique par la taille pour éliminer les zones de petites tailles et conserver les grandes zones de vitrinite. On effectue un filtrage par érosion de manière à ne retenir que les zones de vitrinite homogènes de grande surface. On obtient une image binaire telle que représentée en bas de la figure 4.

Pour déterminer l'image binaire à partir des gradients, on peut par exemple choisir une valeur de gradient égale à 18 ; si le gradient d'un point-image est inférieur ou égal à cette valeur, sa valeur binaire est 1 ; si le gradient d'un point-image est supérieur à cette valeur sa valeur binaire est 0.

La phase suivante consiste à mesurer la distribution des niveaux de gris des zones de vitrinite qui ont été ainsi déterminées. On utilise à cet effet l'image numérisée initiale après correction de l'homogénéité, la mesure n'étant faite que sur les zones de vitrinite qui ont été définies ci-dessus.

Pour obtenir le pouvoir réflecteur de vitrinite on peut utiliser deux méthodes. La première consiste à mesurer le niveau de gris moyens pour l'ensemble des zones de vitrinite de l'échantillon ; une deuxième méthode consiste à déterminer le niveau de gris moyen de chaque zone.

Dans le cas où l'on a à faire à un mélange ou à une pollution, la deuxième méthode permet de mettre facilement en évidence la présence de deux charbons. La mesure du niveau de gris moyen ne comporte pas d'erreur à condition de pondérer la mesure de chaque zone par un coefficient proportionnel à la surface qu'elle occupe.

La mesure du pouvoir réflecteur de vitrinite et l'analyse macérale sont réalisées simultanément, la durée d'analyse étant inférieure à une heure.

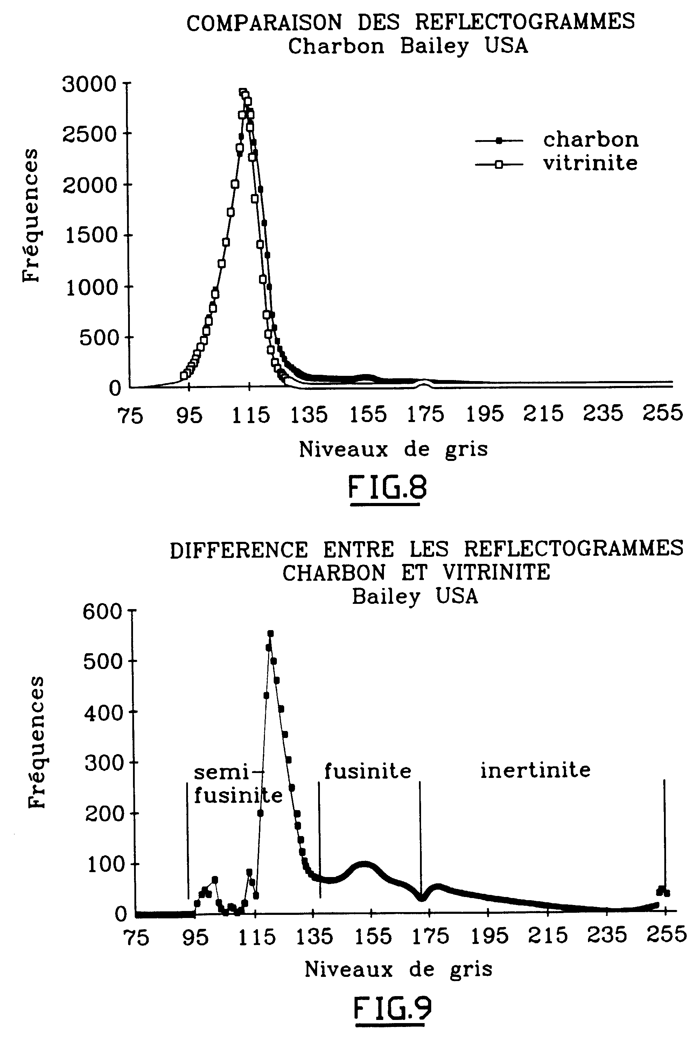

La proportion de vitrinite est estimée à partir de l'intégrale des distributions de la densitométrie globale du charbon et de celle de la vitrinite. Après superposition et par différence, on calcule la proportion de vitrinite. On estime la proportion des autres constituants du charbon par analyse de la distribution sans vitrinite comme cela est illustré sur la figure 9.

Les différentes données obtenues sont enregistrées dans des banques de données, ce qui permet en particulier d'effectuer une surveillance de la qualité des charbons utilisés pour la fabrication de coke.

La demanderesse a effectué des comparaisons entre les résultats fournis par le procédé selon l'invention et ceux fournis par la méthode manuelle de type connu. La comparaison de ces deux méthodes est illustrée sur les figures 5 à 7.

Les figures 5 et 6 sont des courbes de distribution en fonction du pouvoir réflecteur mise sous forme d'histogramme. Les barres noires correspondent à la mesure manuelle de type connu, les barres blanches à la mesure automatique selon l'invention. On peut s'apercevoir qu'il y a une très bonne corrélation entre les mesures effectuées de manière automatique par rapport à la méthode manuelle connue. La figure 5 concerne un charbon de bas rang et la figure 6 un charbon de haut rang.

Cette corrélation est illustrée sur la figure 7 sur laquelle on a représenté en abscisses les mesures du pouvoir réflecteur de la vitrinite obtenues par une méthode manuelle de type connu et en ordonnées les mesures obtenues par le procédé selon l'invention.

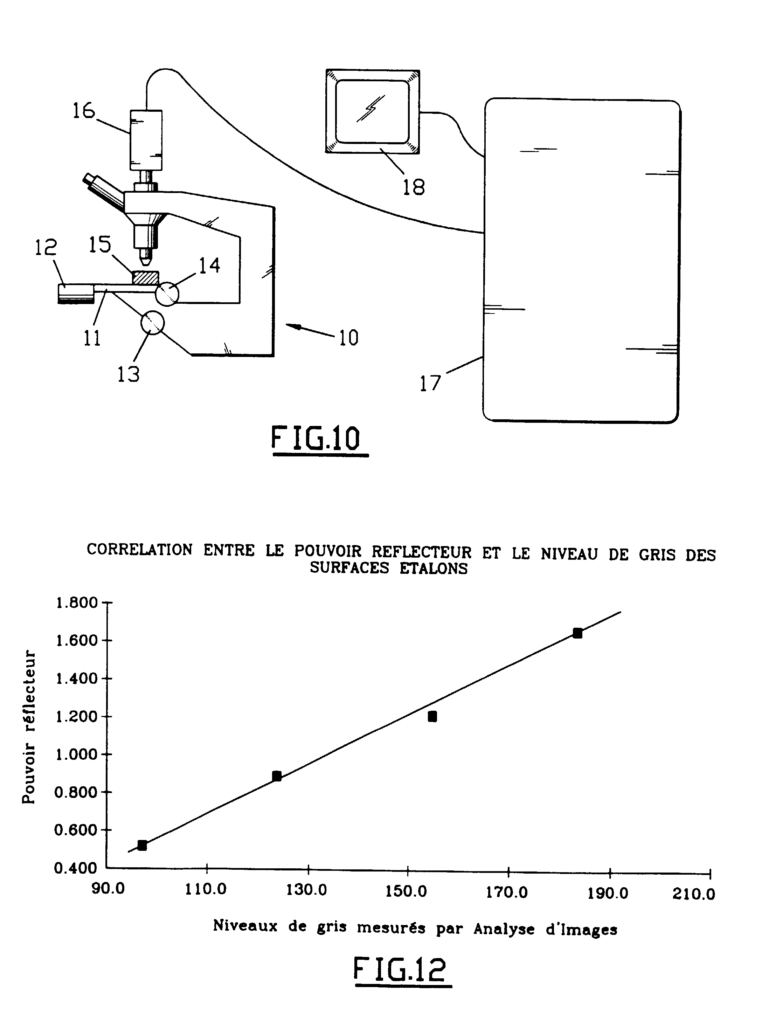

La figure 10 représente, de manière schématique, un dispositif pour la mise en oeuvre de l'invention. Il comporte un microscope optique à lumière réfléchie 10 qui est équipé d'un objectif à immersion d'huile de grossissement 20. Ce microscope comporte une platine 11 qui peut se déplacer selon trois axes (X,Y et Z) au moyen de moteurs 12, 13 et 14. Ceci permet le déplacement programmé de l'échantillon et la mise au point automatique.

Une caméra 16 du type à transfert de charges (CCD) à points formés avec une résolution de 550 x 740 points images est fixée sur le microscope 10.

Les images fournies par la caméra 16 sont envoyées à un système informatique 17 qui comporte en particulier un dispositif de visualisation 18 et qui commande les moteurs du microscope 10.

Les figures 11A à 11C représentent un exemple de traitement d'une plage de charbon.

L'information intéressante dans une plage de charbon est constituée par l'ensemble de l'image complémentaire à la résine d'enrobage apparaissent en noir sur la figure 11A.

Cette résine possède toujours la même gamme de gris comprise entre 0 et 70. L'effet de la porosité est éliminé de la manière qui suit.

Compte tenu de cette remarque, on définit un masque d'analyse correspondant au complémentaire de l'image de la résine érodé de 2,2 $\mu$m. Le but de cette érosion est d'éliminer les défauts de polissage (relief) observé sur le pourtour de la résine et de la porosité. Ce défaut génère des niveaux de gris qui ne correspondent pas à la réalité et que l'on ne prend pas en compte par ce biais.

La figure 3 montre l'effet de ce filtrage sur la distribution des niveaux de gris dans un charbon sans le pic correspondant à la résine et à la porosité.

Toutes les mesures de densitométrie (charbon et vitrinite) sont réalisées à travers ce masque.

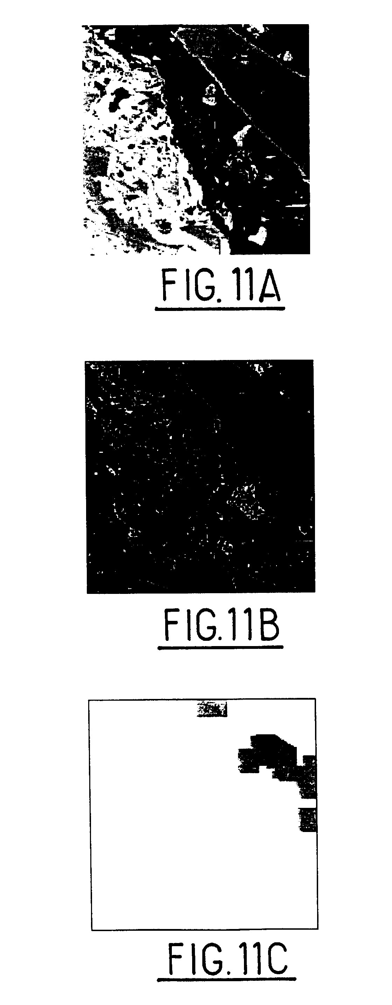

La mise en évidence des plages de vitrinite s'effectue de la manière suivante. Les gradients de gris observés dans cette plage de charbon sont représentés sur la figure 11B. On devine déjà les zones de plus grande homogénéité apparaissant très sombres parce qu'elles sont à faibles gradients.

Le seuillage des gradients à la valeur supérieure à 20 permet l'obtention d'une image binaire ($IB_G$) dont les parties égales à 0 sont les plus homogènes (figure 11B).

Le domaine de réflectance de la vitrinite des charbons cokéfiants est dans les valeurs NG de 80 et 190.

On construit donc un masque binaire ($IB_M$) en prenant uniquement en compte que cette gamme de NG.

On élimine ainsi la porosité, la résine et les inertinites.

La mise en évidence des plages de vitrinite est réalisée en faisant l'intersection de l'image $IB_M$ avec le complémentaire de l'image $IB_G$, l'image résultante est érodée par un élément structurant de 25 $\mu$m puis dilatée de la taille de 20 $\mu$m.

EP 0 657 730 B1

$$((\overline{IB}_G \cap IB_M) \ominus H_{25\mu m}) \oplus H_{20\mu m}$$

Le résultat de ces opérations booléennes est représenté sur la figure 11C.

Les figures 8 et 9 illustrent la manière dont on détermine la composition de l'échantillon de charbon.

Lorsque l'on analyse la densitométrie déterminée sur l'ensemble de l'échantillon, il est impossible d'indiquer sur la distribution la part des niveaux de gris correspondant à la vitrinite.

En discriminant la vitrinite du charbon par le gradient et le filtre morphologique, on peut donc mesurer la distribution des niveaux de gris de la vitrinite sans biais. En déduisant la distribution des niveaux de gris de la vitrinite de celle du charbon, on peut estimer la part des autres macéraux.

L'intégrale de la densitométrie du charbon représente la somme des macéraux. L'intégrale de la densitométrie de la vitrinite représente la part de la vitrinite et l'intégrale de la différence des ces deux distributions donne la part des autres macéraux et permet, en particulier d'estimer la proportion d'inertes.

La figure 12 représente la courbe d'étalonnage, c'est-à-dire la corrélation entre le pouvoir réflecteur et le niveau de gris des surfaces étalons. Ces surfaces étalons, qui sont utilisées pour le procédé d'analyses manuelles, correspond à un pouvoir réflecteur connu (0,525; 0,892; 1,236 et 1,688%).

Les conditions opératoires ont été optimisées pour obtenir une dynamique de niveaux de gris suffisante entre le pouvoir réflecteur le plus bas et le pouvoir réflecteur le plus haut.

On a déterminé la corrélation entre le pouvoir réflecteur et le niveau de gris moyen des surfaces étalons pour vérifier la linéarité de la relation et pour évaluer le niveau de dynamique Nd (figure 5).

$$Nd = \frac{\text{étalon}_{1,688} - \text{étalon}_{0,525}}{NG_{1,688} - NG_{0,525}} = \frac{1,688 - 0,525}{183 - 97} = 0,0135$$

$NG_{1,688}$ = niveau de gris moyen mesuré sur l'étalon 1,688
$NG_{0,525}$ = niveau de gris moyen mesuré sur l'étalon 0,525

Le niveau de dynamique moyen est égal à un pouvoir réflecteur de 0,0135% et se définit comme la valeur moyenne du pouvoir réflecteur pour une unité de niveau de gris.

La valeur de Nd détermine le niveau de sensibilité des mesures. La norme établie par les pétrographes admet un écart absolu de pouvoir réflecteur de plus ou moins 0,05% de pouvoir réflecteur, cette valeur correspond à trois niveaux de gris ce qui est très satisfaisant.

La linéarité n'est pas excellente pour les valeurs de pouvoir réflecteur comprises entre 0,892 et 1,688%, ce domaine de pouvoir réflecteur de la vitrinite correspond à la plus grande partie des charbons utilisés.

Compte tenu de cette remarque la relation entre les deux méthodes est réalisée avec plus de rigueur en appliquant les coefficients de corrélation calculés pour chaque intervalle de pouvoir réflecteur.

Les conditions opératoires qui ont permis d'obtenir ce résultat sont optimum et sont imposées pour chaque analyse de charbon.

Les expériences faites ont permis de constater que le procédé automatique selon l'invention d'analyses macérales des charbons permet de réaliser un gain considérable sur la durée de l'analyse en la réduisant de 4 à 1 heure en moyenne.

Par ailleurs, la précision est nettement améliorée surtout en ce qui concerne l'analyse macérale.

L'invention permet donc d'effectuer un meilleur contrôle des produits dans le but de contribuer à l'élaboration d'un coke de qualité constante et à l'évaluation du prix d'achat des charbons. Il en résulte un avantage économique important.

Enfin, il s'agit d'une méthode non contraignante pour l'opérateur, ce qui explique en particulier l'amélioration de la précision.

## Revendications

1. Procédé d'analyse macérale du charbon en vue de déterminer sa teneur en vitrinite, dans lequel on utilise un échantillon constitué par du charbon broyé enrobé dans de la résine, on explore la surface de l'échantillon au moyen d'un microscope en déplaçant l'échantillon pour obtenir des champs successifs dans le microscope et l'on mesure le pouvoir réflecteur de la vitrinite, caractérisé par les étapes suivantes:

   - pour chaque champ du microscope, on réalise une image numérisée à niveaux de gris ;

- sur chaque image, on effectue une mesure de seuillage pour éliminer les champs contenant une quantité de résine supérieure à un seuil donné ;
- pour chaque champ non éliminé, on effectue l'analyse suivante :

a) on détermine la distribution des niveaux de gris ;
b) on détermine les gradients de niveaux de gris;
c) on réalise une image binaire par seuillage des gradients de gris de manière à déterminer des zones de vitrinite ;
d) on détermine la distribution des niveaux de gris ou densitométrie des zones de vitrinite obtenues ; et
e) on calcule le pouvoir réflecteur de la vitrinite.

2. Procédé d'analyse macérale du charbon selon la revendication 1, caractérisé en ce que le pouvoir réflecteur de la vitrinite est calculé en déterminant le niveau de gris moyen pour l'ensemble des zones de vitrinite de l'échantillon.

3. Procédé d'analyse macérale du charbon selon la revendication 1, caractérisé en ce que le pouvoir réflecteur de la vitrinite est calculé en déterminant le niveau de gris moyen de chaque zone de vitrinite.

4. Procédé d'analyse macérale du charbon selon la revendication 1, caractérisé en ce que l'on effectue un filtrage morphologique par la taille pour éliminer les zones de vitrinite de petite taille avant l'étape de mesure de la distribution des niveaux de gris.

5. Procédé d'analyse macérale du charbon selon la revendication 1, caractérisé en ce que les valeurs du pouvoir réflecteur de la vitrinite sont obtenues à partir des valeurs de densitométrie.

6. Procédé d'analyse macérale du charbon selon la revendication 1, caractérisé en ce que, pour chaque champ non éliminé, on effectue une correction pour chaque point-image en utilisant une image de référence uniforme en niveaux de gris.

7. Procédé d'analyse macérale du charbon selon la revendication 1, caractérisé en ce que l'on effectue un réglage préalable de l'éclairage du microscope au moyen d'une surface étalon dont le pouvoir réflecteur est connu.

8. Procédé d'analyse macérale du charbon selon la revendication 1, caractérisé en ce qu'après le calcul du pouvoir réflecteur de la vitrinite, on calcule la teneur en vitrinite et on estime la teneur des autres composants par analyse de la densitométrie sans vitrinite.

**Patentansprüche**

1. Verfahren zur Maceralanalyse von Kohle hinsichtlich der Bestimmung seines Vitrinitgehaltes, in dem eine Probe verwendet wird, die durch zermahlenen, in Harz eingebettete Kohle gebildet wurde, die Oberfläche der Probe mittels eines Mikroskopes unter Verschieben der Probe untersucht wird, um die aufeinanderfolgenden Gebiete in dem Mikroskop zu erreichen, und das Reflexionsvermögen des Vitrinits gemessen wird, gekennzeichnet durch die folgenden Schritte:

- für jedes Gebiet des Mikroskopes wird ein digitales Graustufenbild aufgenommen;
- auf jedem Bild wird ein Schwellwertverfahren durchgeführt, um die Gebiete zu entfernen, die eine Harzmenge enthalten, die einen gegebenen Schwellwert übersteigt;
- für jedes nicht-entfernte Gebiet wird die folgende Analyse durchgeführt:

a) Bestimmen der Graustufenverteilung;
b) Bestimmen des Graustufengradienten;
c) Aufnehmen eines binären Bildes durch das Schwellwertverfahren der Graugradienten, so daß die Vitrinitzonen bestimmt werden;
d) Bestimmen der Verteilung der Graustufen oder Messung der Schwärze der erhaltenen Vitrinitzonen und
e) Berechnen des Reflexionsvermögens des Vitrinits.

2. Verfahren zur Maceralanalyse von Kohle gemäß Anspruch 1, dadurch gekennzeichnet, daß das Reflexionsvermögen des Vitrinits durch Bestimmen der mittleren Graustufe in bezug auf die Gesamtheit der Vitrinitzonen der Probe berechnet wird.

3. Verfahren zur Maceralanalyse von Kohle gemäß Anspruch 1, dadurch gekennzeichnet, daß das Reflexionsvermögen des Vitrinits durch Bestimmen der mittleren Graustufe jeder Vitrinitzone berechnet wird.

4. Verfahren zur Maceralanalyse von Kohle gemäß Anspruch 1, dadurch gekennzeichnet, daß eine morphologische Filtration nach der Größe durchführt wird, um die Vitrinitzonen kleinerer Größe vor dem Schritt der Messung der Verteilung der Graustufen zu entfernen.

5. Verfahren zur Maceralanalyse von Kohle gemäß Anspruch 1, dadurch gekennzeichnet, daß die Werte des Reflexionsvermögens aus den Werten der Schwärzungsmessung erhalten werden.

6. Verfahren zur Maceralanalyse von Kohle gemäß Anspruch 1, dadurch gekennzeichnet, daß für jedes nicht-entfernte Gebiet eine Korrektur für jeden Bildpunkt unter Verwendung eines in den Graustufen einheitlichen Referenzbildes durchgeführt wird.

7. Verfahren zur Maceralanalyse von Kohle gemäß Anspruch 1, dadurch gekennzeichnet, daß die vorhergende Einstellung der Mikroskopbeleuchtung mittels einer Standardoberfläche durchgeführt wird, deren Reflexionsvermögen bekannt ist.

8. Verfahren zur Maceralanalyse von Kohle gemäß Anspruch 1, dadurch gekennzeichnet, daß nach der Berechnung des Reflexionsvermögens des Vitrinits der Vitrinitgehalt berechnet und der Gehalt der anderen Komponenten durch Schwärzungsmessung ohne Vitrinit geschätzt wird.

## Claims

1. Process for the maceral analysis of coal with a view to determining its vitrinite content, wherein a sample is used consisting of ground coal coated in resin, the surface of the sample is scanned using a microscope by displacing the sample to obtain successive fields in the microscope and the reflectance of the vitrinite is measured, characterised by the following steps:

   - for each field of the microscope, an image is produced which is digitized in levels of grey;
   - on each image, a threshold measurement is taken to eliminate fields containing a quantity of resin exceeding a given threshold;
   - for each field which is not eliminated, the following analysis is carried out:

      a) the distribution of the levels of grey is determined;
      b) the gradients of the levels of grey are determined;
      c) a binary image is obtained by thresholding the gradients of grey so as to determine the vitrinite zones;
      d) the distribution of the levels of grey or the densitometry of the vitrinite zones obtained is determined; and
      e) the reflectance of the vitrinite is calculated.

2. Process for the maceral analysis of coal according to claim 1, characterised in that the reflectance of the vitrinite is calculated by determining the mean level of grey for all the vitrinite zones in the sample.

3. Process for the maceral analysis of coal according to claim 1, characterised in that the reflectance of the vitrinite is calculated by determining the mean level of grey of each vitrinite zone.

4. Process for the maceral analysis of coal according to claim 1, characterised in that morphological filtering by size is carried out to eliminate small-size vitrinite zones before the step of measuring the distribution of the levels of grey.

5. Process for the maceral analysis of coal according to claim 1, characterised in that the reflectance values of the vitrinite are obtained from the densitometry values.

6. Process for the maceral analysis of coal according to claim 1, characterised in that, for each field which is not eliminated, a correction is made for each image point using a reference image uniform in levels of grey.

7. Process for the maceral analysis of coal according to claim 1, characterised in that the lighting of the microscope is adjusted beforehand by means of a calibrating surface of known reflectance.

8. Process for the maceral analysis of coal according to claim 1, characterised in that after the reflectance of the vitrinite has been calculated, the vitrinite content is calculated and the content of the other components is estimated by analysis of the densitometry without vitrinite.

0.5    1         2         3

B          A

## FIG.1

2

1

## FIG.4

Distribution

Pouvoir Réflecteur

## FIG.2

FIG.3

FIG.7

REFLECTOGRAMME DE LA VITRINITE
CHARBON BAILEY – USA

FIG.5

Fréquence en %

mesure automatique
mesure manuelle

Pouvoir réflecteur par classes

REFLECTOGRAMME DE LA VITRINITE
CHARBON PINACLE – USA

FIG.6

Fréquence en %

mesure automatique
mesure manuelle

Pouvoir réflecteur par classes

## COMPARAISON DES REFLECTOGRAMMES
Charbon Bailey USA

FIG.8

## DIFFERENCE ENTRE LES REFLECTOGRAMMES
CHARBON ET VITRINITE
Bailey USA

FIG.9

# FIG.10

CORRELATION ENTRE LE POUVOIR REFLECTEUR ET LE NIVEAU DE GRIS DES
SURFACES ETALONS

Niveaux de gris mesurés par Analyse d'Images

# FIG.12

FIG. 11A

FIG.11B

FIG.11C